# FASCICULE DE BREVET EUROPEEN

(11) **EP 0 408 477 B1**
(45) Date de publication et mention de la délivrance du brevet: **18.01.1995**
(21) Numéro de dépôt: 90420328.8
(22) Date de dépôt: 10.07.1990
(51) Int. Cl.: A61B 17/58

(54) **Dispositif de verrouillage supérieur des broches centromédullaires utilisées pour l'ostéosynthèse des fractures du fémur, du tibia et de l'humérus**
Einrichtung zur Blockierung der oberen Enden von Markraumnägeln, die für die Osteosynthese von Femur-, Tibia- und Humerusfrakturen benötigt werden
Apparatus for superior end fixation of intramedullary nails used for the osteosynthesis of femoral, tibial and humeral fractures

(30) Priorité: 10.07.1989 FR 8909258
(43) Date de publication de la demande: 16.01.1991
(73) Titulaire: FIXANO SA, F-01000 Bourg en Bresse (FR)
(72) Inventeur: De la Caffiniere, Jean-Yves, F-75006 Paris (FR)
(74) Mandataire: Maureau, Philippe

(56) Documents cités:
- EP-A- 0 140 790
- WO-A-81/01647
- DE-U- 8 809 715
- FR-A- 2 512 340
- FR-A- 2 614 781
- FR-A- 2 636 226
- US-A- 2 725 053

## Description

La présente invention concerne un dispositif d'ostéosynthèse pour fractures du fémur, du tibia et de l'humérus, notamment de verrouillage supérieur des broches centro-médullaires utilisées pour l'ostéosynthèse des fractures du fémur, du tibia et de l'humérus.

Il est habituel d'utiliser des broches pour l'ostéosynthèse des fractures diaphysaires, mais ce procédé est insuffisant pour maîtriser les contraintes en rotation ou verticales qui s'exercent au niveau de la fracture.

La présente invention vise à remédier à cet inconvénient en fournissant un dispositif apte à assurer une parfaite immobilisation des parties du membre fracturé.

A cette fin, le dispositif qu'elle concerne, qui est du type décrit par le modèle d'utilité allemand 88 09 715, c'est-à-dire comprenant des broches destinées à être introduites dans le canal médullaire de l'os, immobilisées par rapport à l'os par l'une de leurs extrémités, et une pièce dite de blocage comprenant des canaux pour l'engagement de l'autre extrémité des broches, est caractérisé en ce que, en combinaison :
- la pièce dite "de blocage" a une forme trapézoïdale, comprend des canaux pour l'introduction des broches, dont l'extrémité supérieure de chacun est obturée, et comprend des canaux médians sensiblement perpendiculaires aux canaux précités ;
- des vis pouvant être fixées à l'os au travers de ces canaux médians sont prévues et
- les extrémités des broches situées du côté opposé à celui où se trouve la pièce de blocage sont recourbées.

La fixation des broches aux parties de l'os fracturé est ainsi parfaitement assurée grâce, à une extrémité, à leur forme recourbée, qui les immobilise par rapport à l'os tant suivant leur axe longitudinal qu'en rotation et, à l'autre extrémité, grâce à la forme de la pièce, à son engagement dans l'os et à son verrouillage à celui-ci.

De toute façon, l'invention sera bien comprise à l'aide de la description qui suit, en référence au dessin schématique annexé représentant, à titre d'exemple non limitatif, une forme de réalisation préférée du dispositif selon l'invention.
Figure 1 en est une vue sous deux angles différents, monté sur un tibia ;
Figures 2 à 6 sont des vues à échelle agrandie, sous différents angles et à différents stades de montage, d'éléments qui le composent ;
Figures 7 à 9 sont des vues d'éléments qui le complètent.

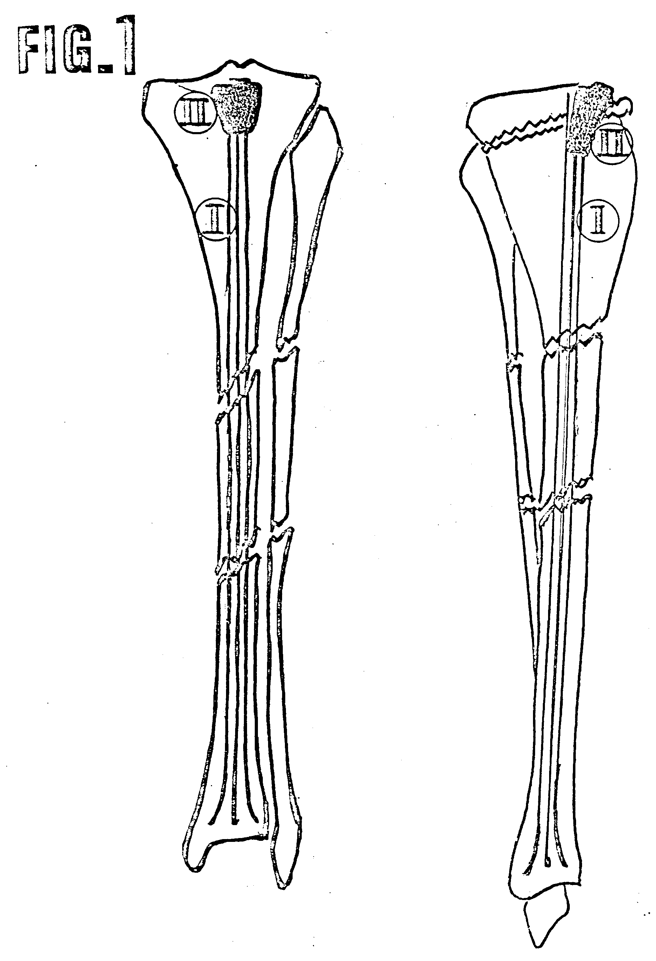

En référence à la figure 1, il apparaît que le dispositif selon l'invention est constitué par une pièce métallique II ayant une forme trapézoïdale et des broches I qui la traversent et dont les extrémités situées du côté opposé à celui où se trouve la pièce II sont recourbées.

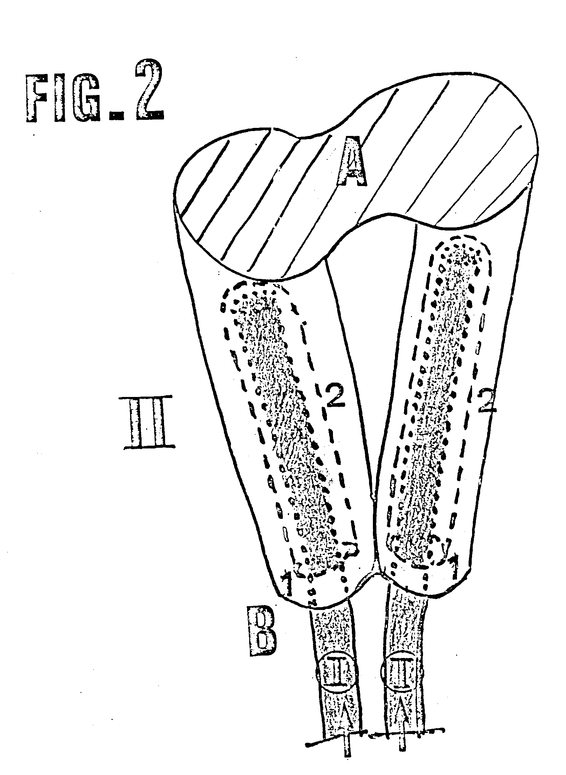

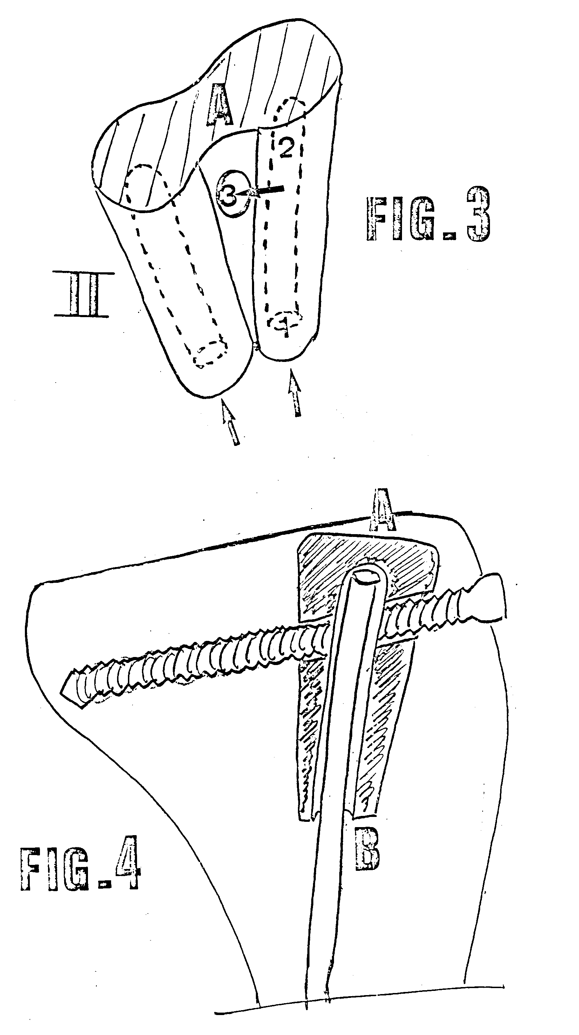

Les figures 2 à 6 représentent plus particulièrement différentes pièces II. Celles-ci sont perforées selon leur grand axe, de deux ou quatre canaux 2 suivant les modèles, débouchant par des orifices 1 dans leur petite base B, de sorte que leur extrémité supérieure est obturée. Les canaux intérieurs 2, qui servent à recevoir chacun l'extrémité supérieure d'une broche I, s'écartent l'un de l'autre depuis leur orifice 1 jusqu'à leur extrémité.

Les pièces II présentent dans leur partie médiane amincie un ou deux trous ou canaux médians 3 d'axe sensiblement perpendiculaire aux canaux 2 précités. Ces canaux ne sont pas représentés sur la figure 2.

Les broches I sont destinées à être introduites dans les canaux 2 tandis que la pièce II est destinée à être introduite dans un logement dont la forme lui correspond, ménagé dans la pièce osseuse.

L'extrémité supérieure obturée de chacun des canaux 2 permet de s'opposer parfaitement aux forces ascendantes et verticales de chacune des broches, ces dernières étant immobilisées par rapport à l'os tant suivant leur axe longitudinal qu'en rotation.

La pièce II est verrouillée à l'os au moyen de vis introduites grâce à un système de visée à travers la pièce osseuse et à travers les canaux 3 de la pièce métallique II.

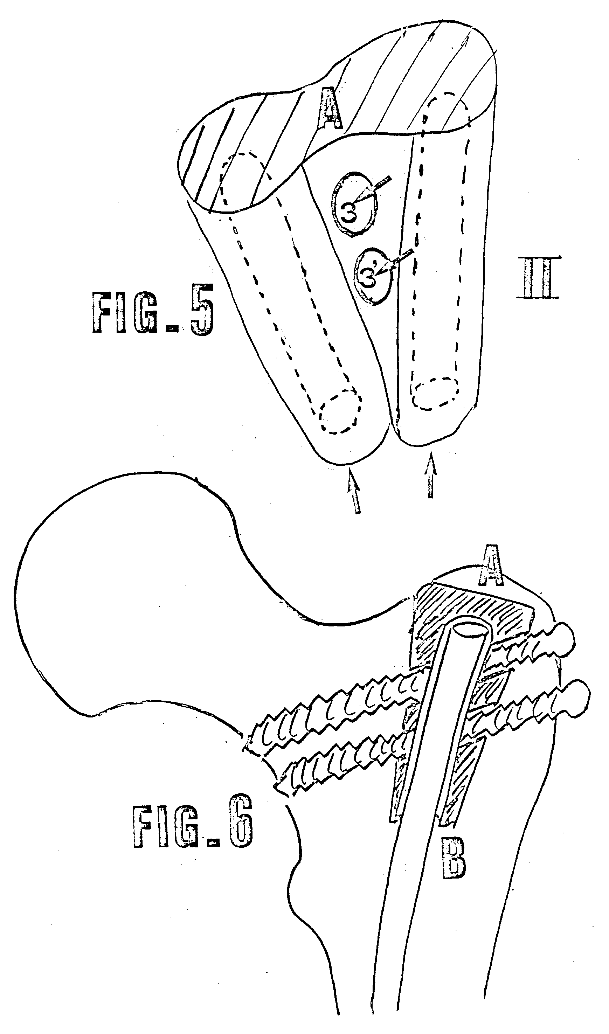

Pour la pièce utilisée avec un tibia (figure 4), ainsi qu'avec un humérus, il n'y a qu'un seul canal 3 ; le blocage est donc assuré par une seule vis (figure 4). Son orientation est de 15° vers le bas par rapport au plan horizontal.

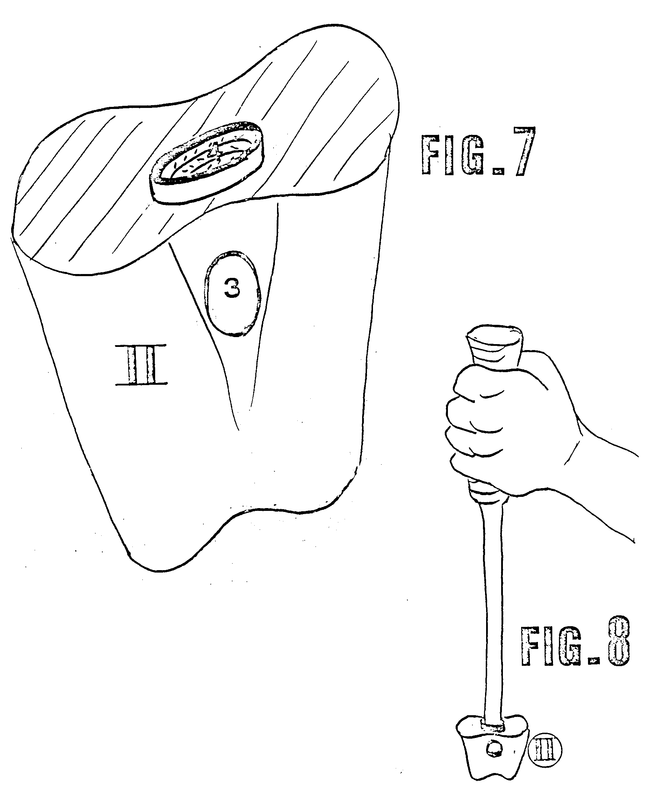

Pour la pièce utilisée avec un fémur (figure 5), il y a deux canaux parallèles 3,3', superposés dans la partie médiane de la pièce II et orientés tous les deux à 40° vers le bas par rapport à un plan horizontal. Ces deux canaux sont suffisamment éloignés l'un de l'autre pour permettre l'introduction indépendante de deux vis de blocage (figure 6).

De plus, les trois variétés de modèles de pièces II diffèrent par leur taille :
- la pièce humérale est la plus petite, elle possède seulement deux canaux verticaux, visant à recevoir deux broches dont le calibre supérieur est de trois millimètres ;
- la pièce tibiale est un peu plus volumineuse, elle peut posséder deux ou quatre canaux verticaux, pouvant recevoir l'extrémité de broches dont le calibre maximum est de quatre millimètres ;
- la pièce fémorale est la plus volumineuse, elle dispose de deux ou quatre canaux verticaux, pouvant recevoir l'extrémité de broches pouvant avoir un calibre de cinq millimètres.

La figure 7 représente un modèle de pièce muni à sa face supérieure d'un dispositif de vissage 4 permettant l'introduction et le vissage de l'extrémité d'un instrument ancillaire visant à porter la pièce pour l'introduire dans l'extrémité osseuse à ostéosynthèser (figure 8) ou fixer le viseur, destiné à la mise en place des vis.

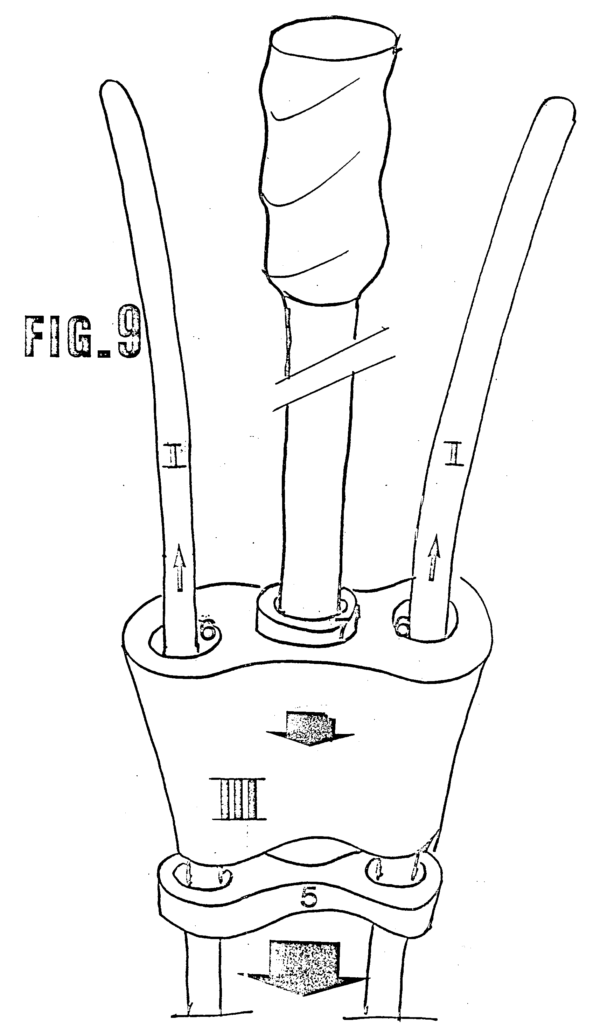

Parmi les instruments ancillaires nécessaires à la mise en place de la pièce de blocage (figure 9), il est prévu une pièce 5 formant un "séparateur de broches" visant à écarter automatiquement les broches pour préparer l'introduction de la pièce définitive de blocage. Ces pièces ont une dimension variable et sont munies de deux, trois ou quatre orifices selon le type de montage. Chaque séparateur a une épaisseur suffisante pour résister aux contraintes en écartement des broches et est introduit de haut en bas après avoir terminé la mise en place des broches centro-médullaires et avant que leur extrémité ne soit sectionnée.

Pour assurer l'écartement de l'extrémité des broches et donc pousser vers le bas le séparateur, le dispositif comprend une pièce dite "d'introduction" III (figure 9), pièce métallique semblable à chacun des modèles de base (fémoral, tibial et huméral), mais dont l'originalité est la suivante : cette pièce d'essai est perforée de part en part de deux ou quatre orifices 6 correspondant aux extrémités des canaux verticaux de la pièce. Ces canaux à orifice d'entrée et de sortie visent à laisser passer les broches mises en place. La pièce est aussi munie à sa face supérieure d'un dispositif de vissage 7 pour y fixer un manche muni d'une poignée, permettant une manipulation aisée. Ce manche est amovible et peut être vissé sur chaque modèle de pièce d'essai.

Comme il est prévu trois modèles de pièces, on prévoiera trois pièces d'essai à deux canaux et éventuellement deux autres modèles à quatre canaux (fémoral et tibial).

Ainsi, la pièce d'essai pourra-t-elle servir à introduire et repousser vers le canal médullaire la pièce séparateur et préparer le site osseux de la pièce de blocage définitive.

## Revendications

1. Dispositif d'ostéosynthèse pour fractures du fémur, du tibia et de l'humérus, comprenant des broches destinées à être introduites dans le canal médullaire de l'os, immobilisées par rapport à l'os par l'une de leurs extrémités, et une pièce dite "de blocage" comprenant des canaux pour l'engagement de l'autre extrémité des broches, caractérisé en ce que, en combinaison :
- la pièce (II) dite "de blocage" a une forme trapézoïdale, comprend des canaux (2) pour l'introduction des broches (I), dont l'extrémité supérieure de chacun est obturée, et comprend des canaux médians (3) sensiblement perpendiculaires aux canaux (2) précités ;
- des vis pouvant être fixées à l'os au travers de ces canaux médians sont prévues et
- les extrémités des broches (I) situées du côté opposé à celui où se trouve la pièce de blocage (II) sont recourbées.

2. Dispositif selon la revendication 1, caractérisé en ce qu'il comprend une pièce (III) dite "d'introduction", de forme semblable à celle de la pièce de blocage (II) et perforée de part en part de deux ou quatre orifices (6) correspondant aux extrémités des canaux verticaux (2) de la pièce (II).

3. Dispositif selon la revendication 1 ou la revendication 2, caractérisé en ce qu'il comprend une pièce (5) destinée à séparer les broches (I) dans laquelle sont ménagés deux à quatre orifices dont les distances qui les séparent entre eux sont égales à celles qui séparent les orifices d'entrée des canaux (2) de chaque type de pièce de blocage (II).

4. Dispositif selon l'une des revendications 1 à 3, caractérisé en ce que les canaux intérieurs (2) s'écartent les uns des autres depuis leur orifice jusqu'à leur extrémité supérieure.

5. Dispositif selon la revendication 1, destiné à l'ostéosynthèse du tibia ou de l'humérus, caractérisé en ce que la pièce (II) de blocage comprend un seul canal (3) orienté vers le bas selon un angle de quinze degrés par rapport à l'horizontale.

6. Dispositif selon la revendication 1, destiné à l'ostéosynthèse du fémur, caractérisé en ce que la pièce (II) de blocage comprend deux canaux (3) parallèles superposés et orientés vers le bas selon un angle de quarante degrés par rapport à l'horizontale.

7. Dispositif selon l'une des revendications 1 à 6, caractérisé en ce que chaque type de pièce (II,III) est muni, à sa face supérieure, d'un dispositif pour le vissage d'un instrument ancillaire.

## Claims

1. Osteosynthesis device for fractures of the femur, tibia and humerus, comprising pins intended to be inserted into the medullary channel of the bone, immobilised with respect to the bone by one of their ends, and a so-called "locking" piece comprising channels for the engagement of the other end of the pins, characterised in that, in combination,
- the so-called "locking" piece (II) has a trapezoidal shape, comprises channels (2) for the insertion of the pins (I), the top end of each of which is closed off, and comprises median channels (3) substantially perpendicular to the aforesaid channels (2);
- screws which can be fixed to the bone through these median channels are provided, and
- the ends of the pins (I) situated on the side opposite to the one where the locking pieces (II) is located are curved.

2. Device according to Claim 1, characterised in that it comprises a so-called "insertion" piece (III), with a shape similar to that of the locking piece (II) and perforated right through by two or four orifices (6) corresponding to the ends of the vertical channels (2) of the piece (II).

3. Device according to Claim 1 or Claim 2, characterised in that it comprises a piece (5) designed to separate the pins (I) in which are provided two to four orifices with the distances separating them from each other being equal to those which separate the entry orifices of the channels (2) of each type of locking piece (II).

4. Device according to one of Claims 1 to 3, characterised in that the inner channels (2) move apart from each other from their orifice to their top end.

5. Device according to Claim 1, intended for osteosynthesis of the tibia or humerus, characterised in that the locking piece (II) comprises a single channel (3) oriented downwards at an angle of fifteen degrees to the horizontal.

6. Device according to Claim 1, intended for osteosynthesis of the femur, characterised in that the locking piece (II) comprises two parallel channels (3) one above the other and oriented downwards at an angle of forty degrees to the horizontal.

7. Device according to one of Claims 1 to 6, characterised in that each type of piece (II, III) has, on its top face, a device for screwing an ancillary instrument.

## Patentansprüche

1. Osteosyntheseeinrichtung für Femur-, Tibia- und Humerusfrakturen, mit in den Markkanal des Knochens einzuführenden Nägeln, die mit Bezug auf den Knochen durch einen ihrer Endbereiche festgelegt sind, und einem sogenannten Blockierstück, das Kanäle für den Eingriff des anderen Endbereiches der Nägel enthält, dadurch gekennzeichnet, daß in Kombination:
- das sogenannte Blockierstück (II) eine trapezförmige Gestalt aufweist, Kanäle (2) zum Einführen der Nägel (I) enthält, deren oberes Ende bei jedem geschlossen ist, und im wesentlichen senkrecht zu den vorgenannten Kanälen (2) verlaufende mittlere Kanäle (3) enthält;
- Schrauben vorgesehen sind, die durch die mittleren Kanäle hindurch am Knochen fixierbar sind, und
- die Endbereiche der Nägel (I), die an der dem Blockierstück (II) entgegengesetzten Seite angeordnet, gebogen sind.

2. Einrichtung nach Anspruch 1, dadurch gekennzeichnet, daß sie ein sogenanntes Einführstück (III) enthält, das eine der Gestalt des Blockierstückes (II) ähnliche Gestalt aufweist und von zwei oder vier Öffnungen (6) ganz durchsetzt ist, die den Endbereichen der vertikalen Kanäle (2) des Stücks (II) entsprechen.

3. Einrichtung nach Anspruch 1 oder 2, dadurch gekennzeichnet, daß sie ein Stück (5) zum Trennen der Nägel (I) enthält, in dem zwei bis vier Öffnungen angeordnet sind, deren sie voneinander trennenden Abstände gleich sind wie diejenigen, die die Eintrittsöffnungen der Kanäle (2) jeden Typs des Blockierstücks (II) trennen.

4. Einrichtung nach einem der Ansprüche 1 bis 3, dadurch gekennzeichnet, daß sich die inneren Kanäle (2) ausgehend von ihrer Mündung bis zu ihrem oberen Ende voneinander entfernen.

5. Einrichtung nach Anspruch 1, bestimmt für die Osteosynthese der Tibia oder des Humerus, dadurch gekennzeichnet, daß das Blockierstück (II) einen einzigen Kanal (3) enthält, der gemäß einem Winkel von fünfzehn Grad mit Bezug auf die Horizontale nach unten gerichtet ist.

6. Einrichtung nach Anspruch 1 , bestimmt für die Osteosynthese des Femur, dadurch gekennzeichnet, das das Blockierstück (II) zwei parallele Kanäle (3) enthält, die übereinanderliegen und gemäß einem Winkel von vierzig Grad mit Bezug auf die Horizontale nach unten gerichtet sind.

7. Einrichtung nach einem der Ansprüche 1 bis 6, dadurch gekennzeichnet, daß jeder Typ des Stücks (II,III) an seiner Oberseite mit einer Einrichtung für das Verschrauben mit einem Hilfsinstrument versehen ist.
